# EUROPEAN PATENT APPLICATION

(11) **EP 2 106 824 A1**
(43) Date of publication of application: **07.10.2009**
(21) Application number: 08703811.3
(22) Date of filing: 24.01.2008
(51) Int. Cl.: A61N 5/06

(54) **OPTICAL BODY HAIR GROWTH REGULATNG DEVICE**

(30) Priority: 25.01.2007 JP 2007014744; 26.01.2007 JP 2007017102
(71) Applicant: Panasonic Electric Works Co., Ltd, Kadoma-shi Osaka 571-8686 (JP)
(72) Inventor: HAMADA, Chosei, Kadoma-shi , Osaka 571-8686 (JP); KINOSHITA, Masato, Kadoma-shi, Osaka 571-8686 (JP); SATOH, Yasuhiro, Kobe-shi, Hyogo 658-0082 (JP)
(74) Representative: Appelt, Christian W.
(86) International application number: PCT/JP2008/050984
(87) International publication number: WO 2008/090953

(57) **Abstract**

A hair growth modulation device is able to detect that a site to be irradiated with modulating light for modulating growth of body hair is a skin in a non-contact manner. This device has a modulating light irradiator 1 for irradiating modulating light, a water content meter 2 for measuring a water content of a target site, and a determinator 3 for judging whether or not to irradiate the modulating light based on the result of the water content meter 2. The water content meter 2 is provided with a measurement light irradiator 6 for irradiating near infrared measurement light having a wavelength of 1300 nm to 2000 nm towards the target site, a light receiving element 7 for receiving light reflected from the target site and converting it to an electric signal, and a signal analyzer 8 for analyzing the electric signal from the light receiving element 7 to obtain a spectrum of the reflected light and to estimate the water content from that spectrum.

## Description

### TECHNICAL FIELD

The present invention relates to a hair growth modulation device for modulating the growth of body hair by irradiating light.

### BACKGROUND ART

Japanese Patent Application Laid-open No. 2002-177405 discloses a laser light irradiating probe for carrying out treatment such as hair removal or hair growth by irradiating laser light to the skin. This laser light irradiating probe has an internal power supply and control circuit that controls the duration of irradiation of laser light with a timer, and is provided with a removal adjuster around the outer periphery of a spherical lens arranged on the head of a case. A contact sensor for detecting contact with the skin surface is provided on the leading end of the adjuster, and allows laser light to only be irradiated when the adjuster is in contact with the skin. As a result, irradiation of laser light from a location away from the skin is prevented, thereby making it possible to prevent an accident, such as the laser light mistakenly entering the eyes, in advance.

However, this laser light irradiating probe lacks convenience in use since it is necessary for a user to keep the leading end of the adjuster in contact with the skin surface at all times. Moreover, when the leading end of the adjuster is in contact with skin around the eyes, for example, there is the possibility of the eyes being irradiated with the laser light, thereby resulting in the risk of damage to the eyes in case of being mistakenly used by a child.

### DISCLOSURE OF THE INVENTION

With the foregoing in view, an object of the present invention is to provide a hair growth modulation device that offers a high degree of convenience in use, and is able to prevent laser light from entering the eyes even in the case of being mistakenly used by a child. The hair growth modulation device as claimed in the present invention has a modulating light irradiator configured to irradiate modulating light for modulating growth of body hair to a target site of a human body, a water content meter configured to measure a water content of the target site of the human body in a non-contact manner, and a determinator configured to judge whether or not to irradiate the modulating light from the modulating light irradiator based on a result of the water content meter. Consequently, whether or not modulating light is irradiated can be determined by estimating whether or not the site to be irradiated is skin without having to contact the target site to be irradiated with modulating light. As a result, modulating light can be safely irradiated even from a location away from the skin, thereby increasing convenience in use. In addition, since it is not necessary to contact a portion of the hair growth modulation device with the skin, the device can be used without any sense of discomfort.

The above-mentioned water content meter is preferably composed of a measurement light irradiator for irradiating measurement light having a wavelength component of 1300 nm to 2000 nm to a target site, a light receiving element for receiving light reflected from the target site and converting it to an electric signal, and a signal analyzer for analyzing the electric signal from the light receiving element to obtain a spectrum of the reflected light and to estimate the water content from that spectrum.

A plurality of the light receiving elements are preferably arranged in a two-dimensional array to measure water content over a wide range.

In addition, a polarizing filter is preferably provided in the measurement light irradiator and the light receiving element so as to capture the reflected light from within the skin as a method for accurately measuring water content of the skin.

Moreover, in the present invention, a supply means is preferably provided for supplying to the target site a liquid substance for lowering the reflectivity of the modulating light. As a result, since irradiation efficiency can be enhanced by lowering the light reflectivity at the target site irradiated with modulating light, the quantity of light required for the light source of the modulating light irradiator can be reduced.

In correlation therewith, the supply means is preferably configured to be enabled only when the determinator judges that the target site is a skin. Consequently, the liquid substance is not supplied to the target site prior to measuring the target site with the measurement light, thereby making it possible to accurately determine whether or not the target site is skin.

### BRIEF DESCRIPTION OF THE DRAWINGS

FIG. 1 is a block diagram showing a hair growth modulation device as claimed in a first embodiment of the present invention;
FIG. 2 is a front view of the hair growth modulation device as claimed in the first embodiment of the present invention;
FIG. 3 is a flow chart indicating the operation of the hair growth modulation device as claimed in the first embodiment of the present invention;

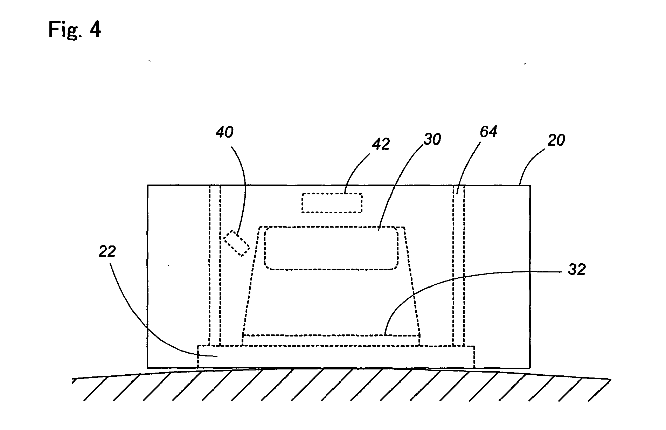

FIG. 4 is a schematic drawing showing a head of the hair growth modulation device as claimed in the first embodiment of the present invention;

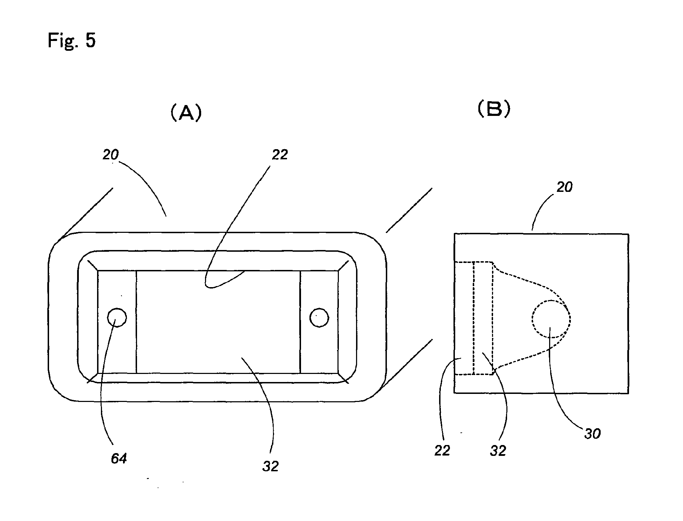

FIGS. 5(A) and 5(B) are a partial exploded view and side view, respectively, of the head of FIG. 4;

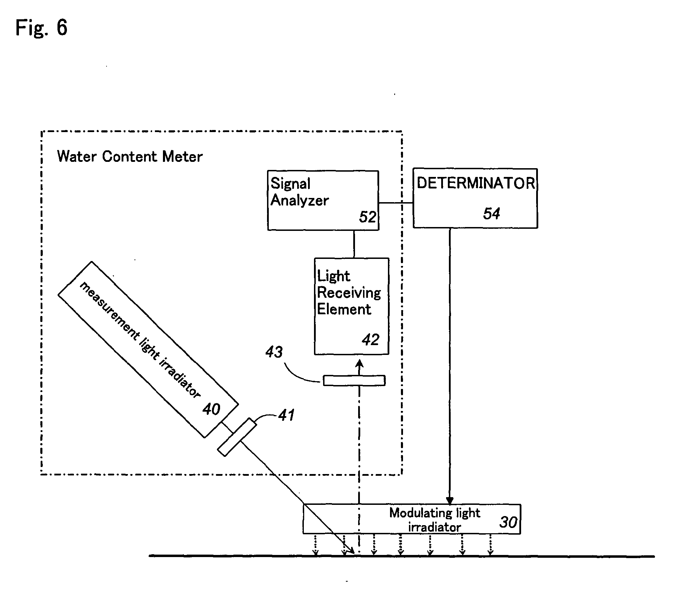

FIG. 6 is a block diagram showing a hair growth modulation device as claimed in a second embodiment of the present invention;
FIG. 7 is a graph indicating spectral reflectivity as measured in skin samples from three persons; and
FIG. 8 is a graph indicating the absorbance of melanin alone.

### BEST MODE FOR CARRYING OUT THE INVENTION

The following provides an explanation of the invention of the present application based on embodiments shown in the appended drawings.

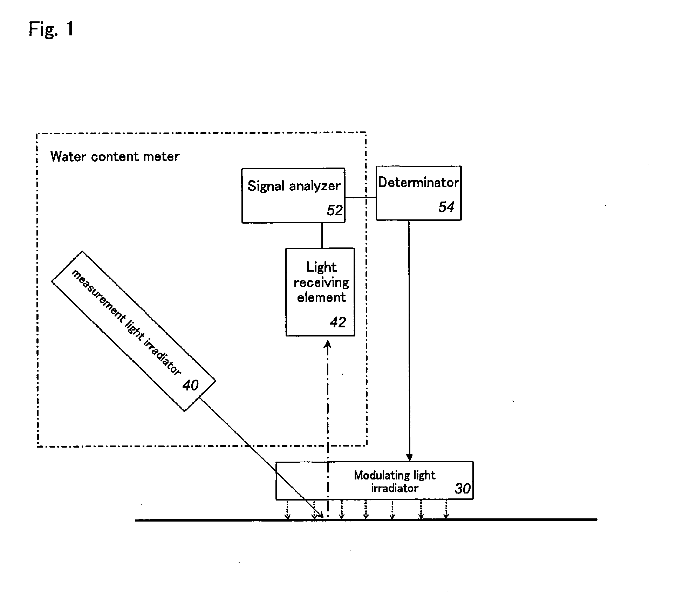

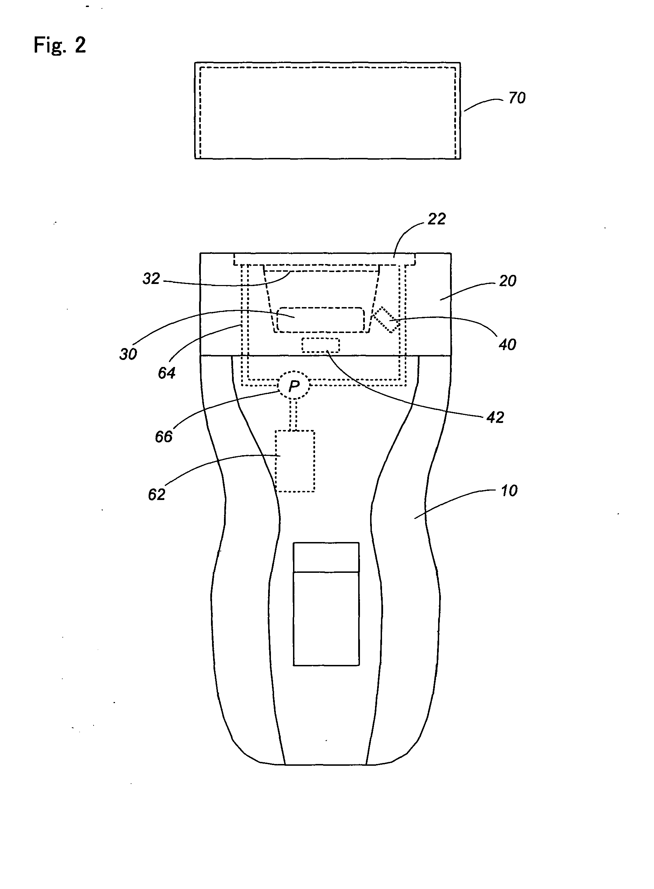

FIGS. 1 and 2 show a hair growth modulation device as claimed in a first embodiment of the present invention. The hair growth modulation device is composed of a grip 10 that is grabbed by a hand of user, a head 20 formed on the upper end of the grip, and a removable cap 70 on the head 20, and a modulating light irradiator 30 is arranged within the head 20. This modulating light irradiator 30 has a xenon flash lamp for the light source, and outputs modulating light in the form of flashing light having a wavelength of 400 to 600 nm to a target site on the skin of a human body to modulate the growth of body hair. Consequently, the modulating light irradiator 30 is provided with a filter and a drive circuit for driving the xenon flash lamp either with a one-shot pulse or continuous pulse and causing the xenon flash lamp to emit modulating light in a short period of time of 1 ms or less. This modulating light is irradiated through a lens 32 formed on a bottom wall of an recess 22 formed on the leading end of the head 20.

The head 20 further houses a measurement light irradiator 40 that irradiates measurement light for measuring water content of a target site, and a light receiving element 42 that receives measurement light reflected from a target site of the human body. The light receiving element 42 outputs an electric signal corresponding to the received light to a control circuit (not shown) housed within the grip 10. This control circuit has a signal analyzer 52 and a determinator 54 realized on a circuit board arranged within the grip 10. The measurement light irradiator 40, the light receiving element 42 and the signal analyzer 52 compose a water content meter for measuring the water content of a target site. The determinator 54 judges whether or not a target site is skin based on the measured water content, and when the target site has been judged to be skin, permits irradiation of modulating light from the modulating light irradiator 30, and when the target site has been judged to not be skin, prohibits irradiation of the modulating light.

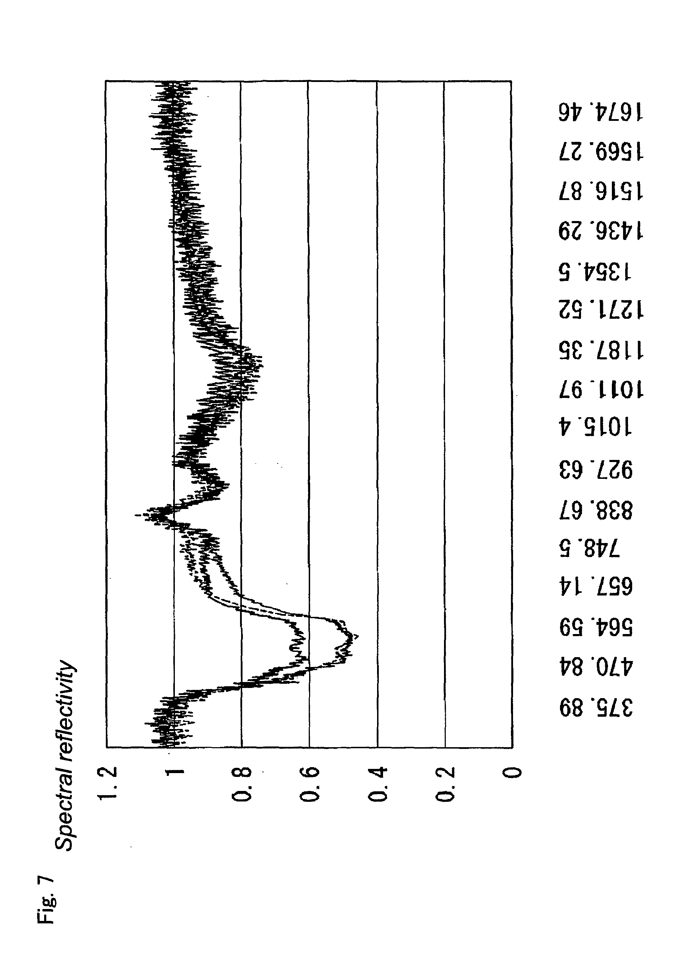

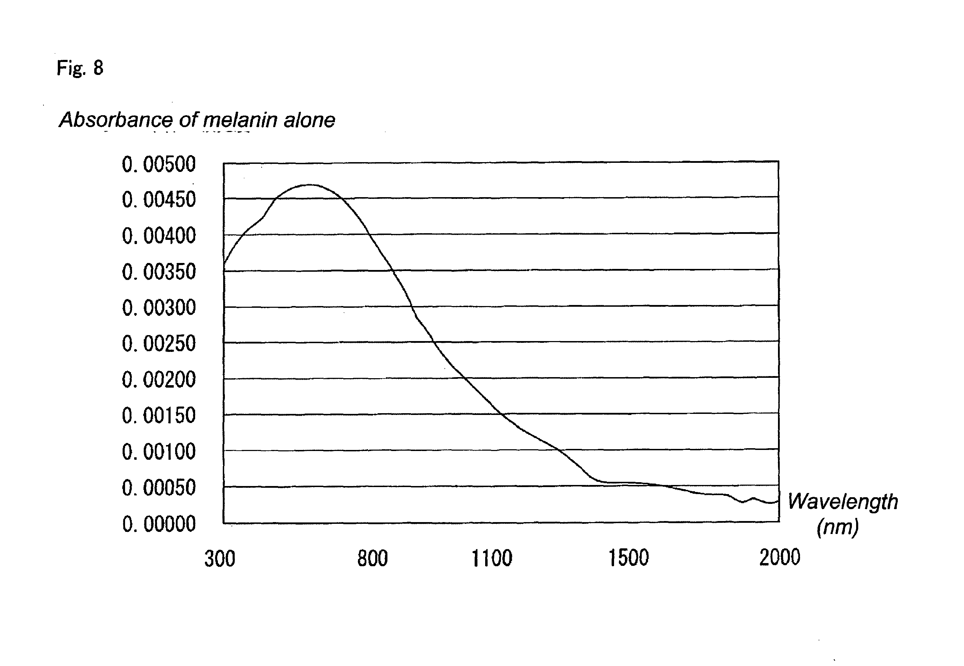

Modulating light irradiated from the modulating light irradiator 1 is required to be housed in the skin, and when selecting the wavelength thereof, spectral reflectance of the skin was measured at each wavelength of light using samples from three Japanese persons as shown in FIG. 7. Since spectral reflectance in the vicinity of 400 to 600 nm decreased for all samples, light in this wavelength range was determined to have the property of being easily absorbed. This is due to the considerable effects of melanin present in skin. FIG. 8 indicates the spectral properties of the absorbance of melanin alone. Melanin alone absorbs 39% of irradiated light in the case of irradiated light having a wavelength of 567 nm. On the basis thereof, the use of light having a wavelength within the range of 400 to 600 nm that is easily absorbed by the skin for the modulating light make it possible to stimulate hair with light at a low output. Furthermore, ultraviolet light having a wavelength of 400 nm or less that is harmful to the skin is cut out with a UV cutoff filter. Furthermore, the modulating light used in the present invention is not necessarily limited to the wavelength range described above, but rather modulating light can be used having a wavelength range of, for example, 400 to 1000 nm.

The measurement light irradiator 40 is configured to irradiate measurement light in the near infrared range having a wavelength of 1300 nm to 2000 nm. The light receiving element 42 receives light reflected from a target site, converts that light to an electric signal, and outputs the electric signal to the signal analyzer 52, after which the electric signal is analyzed by the signal analyzer 52 to determine the spectrum of the reflected light followed by estimation of the water content from that spectrum based on a method to be described later. The incident axis of reflected light that the light receiving element 42 receives is inclined with respect to the measurement light irradiator to the target side at an angle of 45 degrees.

In this manner, since water content can be measured from the reflectivity of near infrared light by irradiating measurement light in the near infrared range to skin of a human body containing moisture, a target site can be measured in a non-contact manner. In addition, since measurement light irradiated from the measurement light irradiator 40 enables water content to be measured using low power light, even if the measurement light is mistakenly irradiated to the eyes, there is no damage thereto. Thus, water content can be measured in a non-contact manner, and safety can be ensured even in the case of the device being mistakenly used by a child.

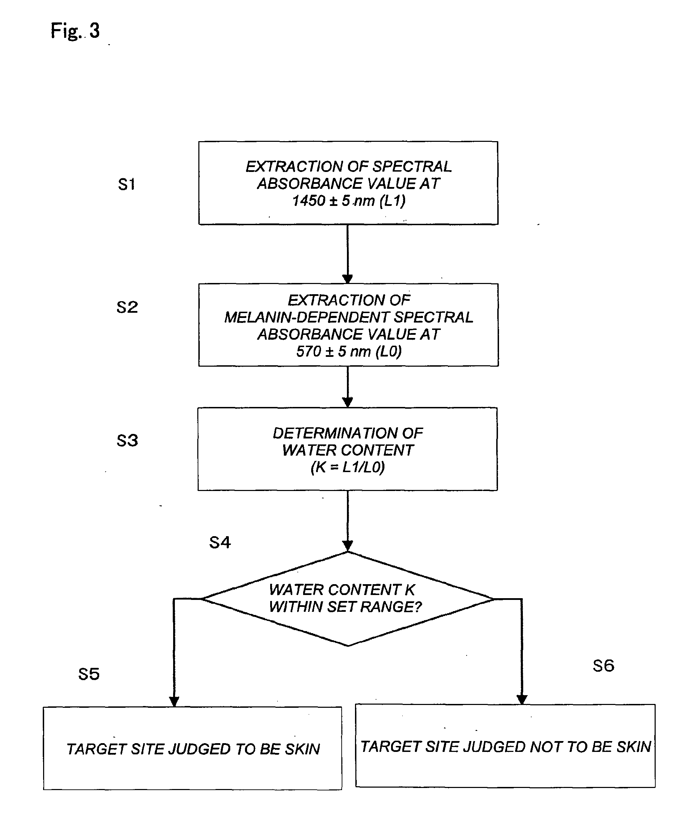

FIG. 3 shows a flow chart of judging whether or not modulating light is to be irradiated from the modulating light irradiator 30.

First, a spectral absorbance value (L1) in the wavelength range which is absorbed by the water. More specifically, the measurement light irradiator 40 irradiates light containing a wavelength component of 1300 nm to 2000 nm to skin of a human body, the light receiving element 42 receives light reflected from the skin of the body and converts it to an electric signal, and the signal analyzer 52 analyzes the electric signal from the light receiving element 42 and determines a special absorbance value (L1) in the wavelength region of 1450 ± 5 nm which is absorbed by the moisture.

Next, a melanin-dependent spectral wavelength value (L0) for the wavelength region of 570 ± 5 nm is determined (S2). Subsequently, the signal analyzer 52 calculates the ratio of the spectral absorbance value (L1) for the wavelength region at which moisture is absorbed of 1450 ± 5 nm to the melanin-dependent spectral absorbance value (L0) for the wavelength region of 570 ± 5 nm, and outputs the water content (K) (K = L1/L0) (S3).

Next, the determinator 54 judges whether the water content (K) output by the signal analyzer 52 is a value within a set range (S4). As a result thereof, if the water content (K) is a value within the set range, the target site is judged to be skin of the human body (S5). If the water content (K) is not a value within the set range, the target site is judged to not be skin of the human body (S6).

As a result of going through these steps for confirming safety, modulating light is only irradiated to a target site 5 from the modulating light irradiator 30 in the case the target site is skin. As a result of this procedure, an eye having a different water content than that of skin is distinguished from the skin. Therefore, even if the device is mistakenly used by a child, this configuration makes it possible to reduce risk that the eye receives the intense light which is irradiated from the light irradiator 30

A plurality of the light receiving elements 42 can also be arranged in the form of a two-dimensional array. In the case a plurality of the light receiving elements 42 are arranged in the form of a two-dimensional array, water content can be measured over a wide range. Consequently, whether or not a target site measured for water content is skin of the human body can be estimated over a wider range, thereby further improving safety.

Measurement of water content by a microwave method or a dielectric constant method instead of the water content measurement method described above is also able to demonstrate similar actions and effects.

The device described above is further provided with a supply means for supplying a liquid substance for reducing reflectivity composed of water or a transparent gel to a target site in order to enhance irradiation efficiency of modulating light to the skin. This supply means is provided with a tank 62 for storing the liquid substance, and a pump 66 provided in a supply pathway 64 for supplying the liquid substance to the recess 22 on the leading end of the head 20. The pump 66 is able to operate only when the target site has been judged to be skin by the determinator 54, and the liquid substance is supplied to the recess 22 either automatically or by the selection of a user.

In the case of supplying the liquid substance, the head 20 is pressed against the skin. While in this state, the space between the lens 32 and the skin is filled with the liquid substance supplied to the recess 22, after which modulating light is irradiated from the modulating light irradiator 30. In this case, an optical stimulus can be adequately imparted to the roots of body hairs since the irradiation efficiency of the modulating light to the roots of body hairs is increased as a result of the reflectivity of the skin surface being lowered by the liquid substance. Since the thickness of the liquid substance is limited to a prescribed value or less by the depth of the recess 22, the liquid substance does not cause a decrease in light transmittance.

The liquid substance decreases reflectivity by eliminating a floating layer of air, and as a result thereof, the thickness of this reflectivity reducing agent is only required to be 0.5 mm or less, while the depth of the recess 22 for defining this thickness is set to 0.5 mm or less in consideration thereof.

In the case the liquid substance is a transparent gel, since a peripheral wall surrounding the outer periphery of the recess 22 functions to ensure uniform thickness together with allowing a thin layer of the transparent gel to be applied to the skin, the transparent gel may first be applied by using this peripheral wall followed by irradiating modulating light.

Although water or a transparent gel is indicated for the liquid substance used to reduce reflectivity, other substances may naturally also be used provided they are able to decrease reflectivity of the skin surface. However, those having the action of cooling the skin that becomes warm due to light irradiation are preferable.

Modulating light irradiated from the modulating light irradiator 30 is irradiated at illuminance of 1,500,000 to 7,000,000 lux and a flash duration (half value of peak power) of 100 to 700 µs. Since the quantity of irradiated light can be determined as the product of illuminance and flash duration, this becomes 150 to 4900 lux·s. In addition, irradiation energy (J/cm²) is the product of irradiation power (W) and irradiation time (seconds), and by controlling the irradiation power (W) and irradiation time (seconds) so that the irradiation energy K is, for example, 0.1 J/cm², the occurrence of adverse side effects attributable to irradiation can be reliably suppressed. Accompanying this, when comparing the case of irradiating light once/day at intervals of several days and the case of repeatedly irradiating light once/day for about 5 to 10 consecutive days, the latter allows the use of a lower power light source.

Hair (including body hair and scalp hair) is known to have a hair cycle during which hair changes in cycles consisting of a growth period, a regressive period and a rest period. The inventors of the present invention confirmed in experiments using mice that if light is irradiated to the skin under the above irradiation conditions, hair growth is effectively inhibited, without causing changes in cell morphology as is observed with existing therapeutic lasers and the like and without causing destruction of cells, if the light is irradiated during the growth period of the hair cycle. Furthermore, hair growth during the growth period of the hair cycle has been confirmed to proceed rapidly if light is irradiated during the rest period of the hair cycle. In addition, adverse side effects such as burns have also been confirmed to not occur.

Although the reason why hair growth is inhibited when hair is irradiated with light at a level that does not cause changes in cell morphology during the growth period is not clear, based on the results of analyses at the RNA level, activation of inflammatory cytokines is thought to occur as a result of being irradiated with light, and the resulting inhibition of hair growth is thought to be the result of this activation of inflammatory cytokines.

Furthermore, irradiation power was changed corresponding to the rate of change of spectral reflectance of the skin an irradiated target site based on the spectral reflectance at 500 to 600 nm at which effects occurred that caused changes in the hair cycle. When the reflectance of a specific wavelength serving as a reference is defined as R0, the reflectance of the irradiated skin is defined as R1, and the power of the light source for which activation of inflammatory cytokines was achieved for the skin of R0 is defined as P0, then the irradiation power P can be determined with the formula P = R1/R0 x P0. Since the energy having an effect on the hair cycle is a constant value, in the case spectral reflectance of the skin is high, power should be increased and irradiation time should be shortened.

Furthermore, since the duration of the growth period, regressive period and rest period of the hair cycle varies according to the location such as the scalp, light is irradiated after first determining whether or not the hair cycle is in the growth period for the location where hair growth is desired to be inhibited.

FIG. 3 shows a hair growth modulation device as claimed in a second embodiment of the present invention. The following provides an explanation of only those matters relating to this device that differ from those of the previously described first embodiment. Thus, explanations of those matters in common with the first embodiment are omitted.

The hair growth modulation device as claimed in the present embodiment is provided with polarizing filters 41 and 43 respectively located in front of the measurement light irradiator 40 and the light receiving element 42.

Even though the target for measurement of water content is inside the skin, when light is irradiated from the measurement light irradiator 6, approximately 70% of the irradiated light ends up being reflected by the skin surface. Consequently, the majority of light received by the light receiving element 7 is reflected by the skin surface, thereby making it difficult to accurately measure light reflected from inside the skin. On the other hand, in contrast to light reflected from the skin surface having the same angle of polarization as the irradiated light, light that has passed through the inside of the skin is known to undergo a change in the angle of polarization. Consequently, in this embodiment, the polarizing filter 41 is disposed in front of the measurement light irradiator 40, thereby the light being polarized to a prescribed polarization angle. The polarized light is irradiated to the target site. Water content can be measured accurately by only measurement light reflected from inside the skin by arranging a polarizing filter 43 in front of the light receiving element 42 that only allows transmission of light polarized to the angle of polarization of light reflected from within the skin.

Here, although different light sources are used for the modulating light irradiator 30 and the measurement light irradiator 40 in the embodiments described above, the same light source can also be used for both. As a result thereof, the number of parts can be decreased and costs can be reduced. However, the irradiation power of the irradiated light is set to a low level in the case of irradiating the measurement light, and is set to a high level in the case of irradiating the modulating light.

## Claims

1. A hair growth modulation device comprising:
a modulating light irradiator configured to irradiate a
modulating light for modulation of hair growth to target sites of a human body;
a water content meter configured to measure a water content of the target site in a non-contact manner,
a determinator configured to make a judgment of whether or not to irradiate the modulating light based upon a result of the water content meter.

2. A hair growth modulation device as set forth in claim 1, wherein
said water content meter comprises a measurement light irradiator for irradiating a measuring light having a wavelength of 1300 nm to 2000 nm to the target site,
a light receiving element configured to receive the light reflected from the target site and convert it into an electrical signal; and
a signal analyzer configured to analyze the electric signal from the light receiving element to obtain a spectrum of the reflected light, and to estimate the water content.

3. A hair growth modulation device as set forth in claim 2, wherein
a plurality of said light receiving elements are arranged in a two-dimensional array.

4. A hair growth modulation device as set forth in claim 2 or 3, wherein
said measuring light irradiator and said light receiving element are each provided with a polarizing filter.

5. A hair growth modulation device as set forth in claim 1, further including a supply means for supplying a liquid substance to the target site, said liquid substance lowers reflectivity of the modulating light.

6. A hair growth modulation device as set forth in claim 5, wherein
said supply means is configured to be enabled only when said determinator judges that the target site is a skin.
